# EUROPEAN PATENT SPECIFICATION

(11) **EP 3 883 948 B1**
(45) Date of publication and mention of the grant of the patent: **25.02.2026**
(21) Application number: 20717603.3
(22) Date of filing: 02.04.2020
(51) Int. Cl.: C07J 17/00, A23L 2/60, A23L 27/30

(54) **MOGROSIDE COMPOUNDS AND USES THEREOF**
MOGROSIDVERBINDUNGEN UND VERWENDUNGEN DAVON
COMPOSÉS MOGROSIDES ET LEURS UTILISATIONS

(30) Priority: 04.04.2019 WO PCT/CN2019/081409
(43) Date of publication of application: 29.09.2021
(73) Proprietor: Firmenich SA, 1242 Satigny (CH)
(72) Inventor: GAN, Xian-Wen, SHANGHAI 201108 (CN); YIN, Dan-Ting, SHANGHAI 201108 (CN)
(74) Representative: Strych, Sebastian
(86) International application number: PCT/EP2020/059442
(87) International publication number: WO 2020/201446

(56) References cited:
- WO-A2-2017/075257
- WO-A2-2018/204483
- DATABASE Chemical Abstracts [online] Chemical Abstracts; 1 January 2020 (2020-01-01), GAN XIAN-WEN ET AL: "Mogroside compounds and application as sweeteners", XP093147824, retrieved from STN accession no. 173:676887 Database accession no. 2020:1995894
- DATABASE Chemical Abstracts [online] Chemical Abstracts; 1 January 2017 (2017-01-01), PATRON ANDREW ET AL: "High intensity sweeteners", XP093147833, retrieved from STN accession no. 166:514780 Database accession no. 2017:701790
- DATABASE Chemical Abstracts [online] Chemical Abstracts; 1 January 2018 (2018-01-01), PATRON ANDREW ET AL: "Engineered metabolic pathways for the production of high intensity mogroside sweeteners", XP093147837, retrieved from STN accession no. 169:537417 Database accession no. 2018:2113021
- DATABASE Chemical Abstracts [online] Chemical Abstracts; 1 January 2017 (2017-01-01), LI FU ET AL: "Cucurbitane glycosides from the fruit of Siraitia grosvenori and their effects on glucose uptake in human HepG2 cells in vitro", XP093147839, retrieved from STN accession no. 167:575901 Database accession no. 2017:286669
- KAZUHIRO MATSUMOTO ET AL: "Minor cucurbitane-glycosides from fruits of Siraitia grosvenori (Cucurbitaceae).", CHEMICAL AND PHARMACEUTICAL BULLETIN, vol. 38, no. 7, 1 January 1990 (1990-01-01), JP, pages 2030 - 2032, XP055588849, ISSN: 0009-2363, DOI: 10.1248/cpb.38.2030
- FU LI ET AL: "Cucurbitane glycosides from the fruit of Siraitia grosvenori and their effects on glucose uptake in human HepG2 cells in vitro", FOOD CHEMISTRY, vol. 228, 10 February 2017 (2017-02-10), NL, pages 567 - 573, XP055681030, ISSN: 0308-8146, DOI: 10.1016/j.foodchem.2017.02.018

## Description

### TECHNICAL FIELD

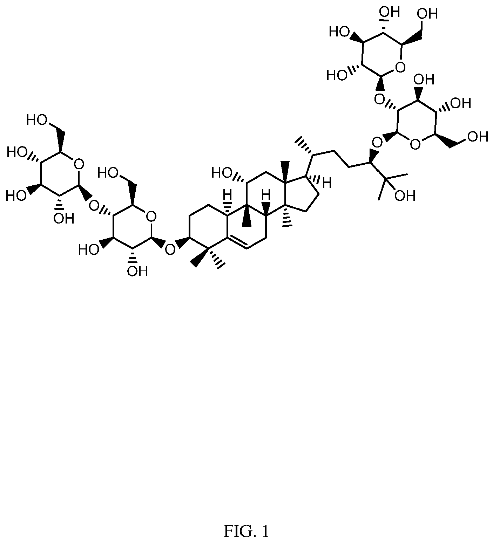

The present disclosure generally relates to various formulations and uses of the compound: Isomogroside IV_{E}, whose chemical formula is shown in FIG. 1, and which is also referred to herein as "MC1". In some aspects, the disclosure provides compositions that include MC1. In some embodiments, the compositions are comestible compositions, including, but not limited to, packaged food and beverage products and tabletop sweeteners. In some aspects, the disclosure provides certain compositions that include such flavanone derivatives, such as compositions that include such flavanone derivatives and one or more other sweeteners. In some other aspects, the disclosure provides methods of reducing the caloric content of a sweetened article, such as a sweetened food or beverage product.

### DESCRIPTION OF RELATED ART

The taste system provides sensory information about the chemical composition of the external world. Taste transduction is one of the more sophisticated forms of chemically triggered sensation in animals. Signaling of taste is found throughout the animal kingdom, from simple metazoans to the most complex of vertebrates. Mammals are believed to have five basic taste modalities: sweet, bitter, sour, salty, and umami.

Sweetness is the taste most commonly perceived when eating foods rich in sugars. Mammals generally perceive sweetness to be a pleasurable sensation, except in excess. Caloric sweeteners, such as sucrose and fructose, are the prototypical examples of sweet substances. Although a variety of no-calorie and low-calorie substitutes exist, these caloric sweeteners are still the predominant means by which comestible products induce the perception of sweetness upon consumption.

Metabolic disorders and related conditions, such as obesity, diabetes, and cardiovascular disease, are major public health concerns throughout the world. And their prevalence is increasing at alarming rates in almost every developed country. Caloric sweeteners are a key contributor to this trend, as they are included in various packaged food and beverage products to make them more palatable to consumers. In many cases, no-calorie or low-calorie substitutes can be used in foods and beverages in place of sucrose or fructose. Even so, these compounds impart sweetness differently from caloric sweeteners, and many consumers fail to view them as suitable alternatives. Moreover, such compounds may be difficult to incorporate into certain products. In some instances, they may be used as partial replacements for caloric sweeteners, but their mere presence can cause many consumers to perceive unpleasant off-tastes including, astringency, bitterness, and metallic and licorice tastes.

WO 2017/075257 A2 discloses high intensity sweeteners.

WO 2018/204483 A2 discloses methods for making high intensity sweeteners.

Li et al. (Food Chemistry, 228 (2017) 567-573) discloses cucurbitane gylcosides from the fruit of Siraitia grosvenori and their effects on glucose uptake in human HepG2 cells in vitro.

Thus, currently available lower-calorie sweeteners face certain challenges to their adoption. Thus, there is a continuing need to discover and develop no-calorie or low-calorie sweeteners that may be more acceptable to consumers.

### SUMMARY

The present disclosure relates to the discovery that Isomogroside IV_{E}, which is also referred to herein as "MC1" and which is represented by the chemical structure 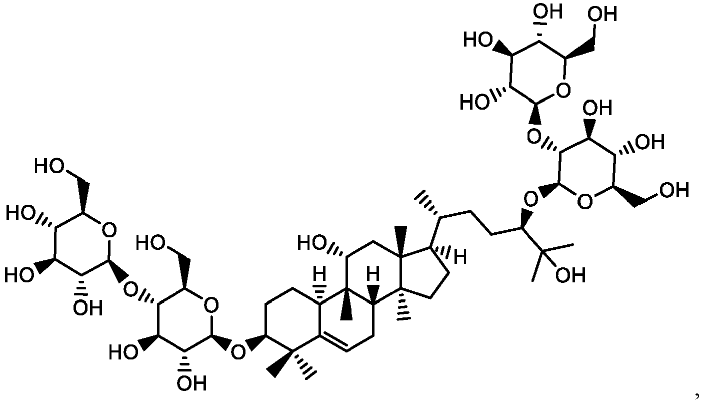 imparts certain beneficial taste properties when used in combination with other sweeteners and flavor-modifying compounds, and when formulated in particular solid-state forms.

In a first aspect, the disclosure provides the use of Isomogroside IV_{E} to sweeten a comestible composition or to enhance the sweetness of a comestible composition.

The disclosure provides the claimed use or composition of solid-state forms, such as crystalline polymorphs, co-crystals, and solvates of the compounds of the first aspect.

In a second aspect, the invention provides formulations of the compounds of the first aspect for use in comestible products, such as food and beverage products, and in combination with other sweeteners, flavor modifiers, mouthfeel enhancers, and the like wherein the mogroside compound is present in the comestible composition at a concentration from 5 to 1000 ppm.

The invention provides uses of the compounds of the first aspect, the solid-state forms of the second aspect, or the formulations of the third aspect to modify the flavor or to impart flavor, such as to impart sweetness, to a comestible product, such as a food or beverage product.

Further aspects, and embodiments thereof, are set forth below in the Detailed Description, the Drawings, the Abstract, and the Claims.

### BRIEF DESCRIPTION OF THE DRAWINGS

The following drawings are provided for purposes of illustrating various embodiments of the compositions and methods disclosed herein. The drawings are provided for illustrative purposes only, and are not intended to describe any preferred compositions or preferred methods, or to serve as a source of any limitations on the scope of the claimed inventions.
FIG. 1 shows a chemical formula that represents the compound Isomogroside IV_{E}.
FIG. 2 shows an LC-HR-MS spectrum of (1S,4R,9beta,11alpha,24R)-1-{[4-O-(beta-D-glucopyranosyl)-beta-D-glucopyranosyl]oxy}-11,25-dihydroxy-9,10,14-trimethyl-4,9-cyclo-9,10-secocholest-5-en-24-yl 2-O-beta-D-glucopyranosyl-beta-D-glucopyranoside (Isomogroside IV_{E}).
FIG. 3 shows a ¹H NMR spectrum of (1S,4R,9beta,11alpha,24R)-1-{[4-O-(beta-D-glucopyranosyl)-beta-D-glucopyranosyl]oxy }-11,25-dihydroxy-9,10,14-trimethyl-4,9-cyclo-9,10-secocholest-5-en-24-yl 2-O-beta-D-glucopyranosyl-beta-D-glucopyranoside (Isomogroside IV_{E}).
FIG. 4 shows a ¹³C NMR spectrum of (1S,4R,9beta,11alpha,24R)-11-{[4-O-(beta-D-glucopyranosyl)-beta-D-glucopyranosyl]oxy}-11,25-dihydroxy-9,10,14-trimethyl-4,9-cyclo-9,10-secocholest-5-en-24-yl 2-O-beta-D-glucopyranosyl-beta-D-glucopyranoside (Isomogroside IV_{E}).

### DETAILED DESCRIPTION

The following Detailed Description sets forth various aspects and embodiments provided herein. The description is to be read from the perspective of the person of ordinary skill in the relevant art. Therefore, information that is well known to such ordinarily skilled artisans is not necessarily included.

### Definitions

The following terms and phrases have the meanings indicated below, unless otherwise provided herein. This disclosure may employ other terms and phrases not expressly defined herein. Such other terms and phrases have the meanings that they would possess within the context of this disclosure to those of ordinary skill in the art. In some instances, a term or phrase may be defined in the singular or plural. In such instances, it is understood that any term in the singular may include its plural counterpart and vice versa, unless expressly indicated to the contrary

As used herein, "solvate" means a compound formed by the interaction of one or more solvent molecules and one or more compounds described herein. In some embodiments, the solvates are comestibly acceptable solvates, such as hydrates.

A "sweetener", "sweet flavoring agent", "sweet flavor entity", or "sweet compound" herein refers to a compound or comestibly acceptable salt thereof that elicits a detectable sweet flavor in a subject, e.g., a compound that activates a T1R2/T1R3 receptor in vitro.

As used herein, the "ppm" refers to a concentration of mg/kg, based on the total weight of the composition.

As used herein, the singular forms "a," "an," and "the" include plural referents unless the context clearly dictates otherwise. For example, reference to "a substituent" encompasses a single substituent as well as two or more substituents, and the like.

As used herein, "for example," "for instance," "such as," or "including" are meant to introduce examples that further clarify more general subject matter. As used herein, "comprise" or "comprises" or "comprising" or "comprised of" refer to groups that are open, meaning that the group can include additional members in addition to those expressly recited. For example, the phrase, "comprises A" means that A must be present, but that other members can be present too. The terms "include," "have," and "composed of" and their grammatical variants have the same meaning. In contrast, "consist of" or "consists of" or "consisting of" refer to groups that are closed. For example, the phrase "consists of A" means that A and only A is present.

As used herein, "optionally" means that the subsequently described event(s) may or may not occur. In some embodiments, the optional event does not occur. In some other embodiments, the optional event does occur one or more times.

As used herein, "or" is to be given its broadest reasonable interpretation, and is not to be limited to an either/or construction. Thus, the phrase "comprising A or B" means that A can be present and not B, or that B is present and not A, or that A and B are both present. Further, if A, for example, defines a class that can have multiple members, e.g., A₁ and A₂, then one or more members of the class can be present concurrently.

Chemical structures are often shown using the "skeletal" format, such that carbon atoms are not explicitly shown, and hydrogen atoms attached to carbon atoms are omitted entirely. For example, the structure 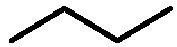 represents butane (i.e., n-butane). Furthermore, aromatic groups, such as benzene, are represented by showing one of the contributing resonance structures. For example, the structure 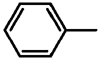 represents toluene.

Other terms are defined in other portions of this description, even though not included in this subsection.

### Mogroside Compound

In a first aspect, the disclosure provides the use of a mogroside compound to sweeten a comestible composition or to enhance the sweetness of a comestible composition, wherein the mogroside compound is a compound of the following formula: 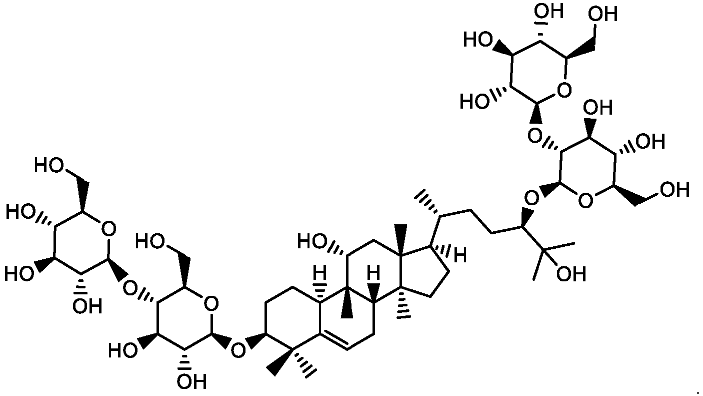 The compound of the above formula is also described herein as (1S,4R,9beta,11alpha,24R)-1-{[4-O-(beta-D-glucopyranosyl)-beta-D-glucopyranosyl]oxy}-11,25-dihydroxy-9,10,14-trimethyl-4,9-cyclo-9,10-secocholest-5-en-24-yl 2-O-beta-D-glucopyranosyl-beta-D-glucopyranoside, or by the abbreviation "MC1".

The sugar moieties shown in the structure above are shown in their closed, cyclic form. The structure also encompasses forms where the depicted sugar moieties are in their open, acyclic form.

Isotopes may be present in the compounds described. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

### Solid State Forms and Solutions of Mogroside Compound

The disclosure provides the claimed use or composition of various solid-state forms of the MC1 compound.

In some embodiments, the MC1 compound exists as a crystalline solid, either in substantially pure form or in a formulation such as those set forth below. The crystalline solid can have any suitable polymorphic form, such as any polymorphic form obtainable via recrystallization in any suitable solvent system, according to techniques commonly used in the art of polymorph screening.

In some other embodiments, the MC1 compound exists as an amorphous solid or a semi-amorphous solid, meaning that it lacks any regular crystalline structure. Such solids can be generated using standard techniques, such as spray drying, and the like.

In some embodiments, the MC1 compound exists as a solvate, which is a pseudomorphic form of the compound in which one or more solvent molecules (such as water molecules) are taken up into the crystalline structure. Any suitable solvent or combination of solvents can be used, including, but not limited to, water, methanol, ethanol, n-propanol, isopropanol, n-butanol, 2-butanol, isobutanol, ethyl acetate, ethylene glycol, 1,2-propylene glycol, 1,3-propylene glycol, and the like. In some embodiments, the disclosure provides hydrates of the MC1 compound. Such solvates can be generated by any suitable means, such as those techniques typically used by skilled artisans in the field of polymorph and solvate screening.

In some other embodiments, the MC1 compound exist as a co-crystal with one or more other compounds, such as one or more other sweetener compounds. The MC1 compound can form a co-crystal with any suitable compound. Non-limiting examples of such suitable compounds include fructose, glucose, galactose, sucrose, lactose, maltose, allulose, sugar alcohols (such as erythritol, sorbitol, xylitol, and the like), sucralose, steviol glycosides (such as rebaudioside A, rebaudioside E, rebaudioside M, and the like natural stevioside compounds), other mogrosides (such as mogroside V, isomogroside V, siamenoside I, isomogroside III_{E}, the 1,6-α isomer of siamenoside I, and the like), aspartame, saccharin, acesulfame K, cyclamate, inulin, isomalt, and maltitol. Such co-crystals can be generated by any suitable means, such as those set forth in U.S. Patent Application Publication No. 2018/0363074.

In some embodiments, the MC1 compound is in the form of a dry particle. Such dry particles can be formed by standard techniques in the art, such as dry granulation, wet granulation, and the like. Such particles can also contain a number of excipients, including, inert diluents, such as calcium carbonate, sodium carbonate, lactose, calcium phosphate, and sodium phosphate; granulating and disintegrating agents, such as starch, cellulosic materials, and alginic acid; binding agents, such as gelatin, guar gum, and acacia; and lubricating agents, such as magnesium stearate, stearic acid, and talc. Other excipients typically used in food and beverage products can also be included, such as typical foodstuff materials.

In some embodiments, the MC1 compound are in the form of a liquid solution or a liquid suspension. Such compositions can also include: carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. Such compositions can also include one or more coloring agents, one or more flavoring agents, and the like. Such liquid suspensions and solutions have a liquid carrier. In general, the liquid carrier comprises water. In some such cases, the liquid composition is an emulsion, such as an oil-in-water or a water-in-oil emulsion. Further, in some cases, water may be too polar to dissolve the MC1 compound to the desired concentration. In such instances, it can be desirable to introduce water-miscible solvents, such as alcohols, glycols, polyols, and the like, to the solvent to enhance solubilization of the MC1 compound.

In some embodiments, the MC1 compound is in the form of a solution, i.e., are solvated within a liquid carrier. In some embodiments, the liquid carrier is an aqueous carrier. Such solutions can be diluted to any suitable concentration.

### Formulations, Uses, and Methods

In another aspect, the invention provides a comestible composition comprises the MC1 compound according to claim 7. The disclosure provides uses of the MC1 compound to sweeten a comestible composition. It is merely disclosed that the disclosure provides uses of the MC1 compound to reduce the bitterness of a comestible composition. The disclosure provides uses of the MC1 compound to enhance the sweetness of the comestible composition. In some embodiments thereof, the comestible composition comprises a another sweetener (according to any of the embodiments set forth below). It is merely disclosed but not part of the invention that the disclosure provides uses of the MC1 compound to enhance the umami taste of a comestible composition. It is merely disclosed but not part of the invention that the disclosure provides uses of the MC1 compound to reduce the sourness of a comestible composition.

The invention also provides methods that correspond to each of the foregoing uses. Thus, in certain related aspects, the invention provides methods of enhancing the sweetness of a comestible composition, comprising introducing an amount (such as a sweetness-enhancing effective amount) of the MC1 compound to the comestible composition. The invention provides methods of sweetening a comestible composition, comprising introducing an amount (such as a sweetening effective amount) of the MC1 compound to the comestible composition. It is merely disclosed but not part of the invention that the disclosure provides methods of reducing the bitterness of a comestible composition, comprising introducing an amount (such as a bitterness-reducing effective amount) of the MC1 compound to the comestible composition.

The foregoing uses and methods generally involve the use of the MC1 compound in a composition containing one or more additional ingredients. For example, in at least one aspect, the disclosure provides compositions comprising the MC1 compound wherein the MC1 compound makes up at least 1% by weight, or at least 2% by weight, or at least 3% by weight, or at least 5% by weight, or at least 10% by weight, or at least 20% by weight, or at least 30% by weight, or at least 40% by weight, of the compositions on a dry weight basis (e.g., based on the total weight of the composition excluding the weight of any liquid carrier). In a related aspect, the disclosure provides solid-state compositions comprising the MC1 compound wherein the MC1 compound make up at least 1% by weight, or at least 2% by weight, or at least 3% by weight, or at least 5% by weight, or at least 10% by weight, or at least 20% by weight, or at least 30% by weight, or at least 40% by weight, of the solid-state compositions, based on the total weight of composition. In another related aspect, the disclosure provides comestible compositions comprising the MC1 compound wherein the concentration of the MC1 compound in the comestible compositions is at least 10 ppm, or at least 15 ppm, or at least 20 ppm, or at least 25 ppm, or at least 30 ppm, or at least 40 ppm, or at least 50 ppm, or at least 75 ppm, or at least 100 ppm, or at least 150 ppm, or at least 200 ppm, or at least 250 ppm, or at least 300 ppm. In another related aspect, the disclosure provides comestible compositions comprising the MC1 compound wherein the comestible compositions comprise another sweetener, such as sucrose, fructose, xylitol, erythritol, allulose, glucose, or combinations thereof. In another related aspect, the disclosure provides a concentrated sweetening composition comprising any mogroside compounds of the foregoing aspects, including any embodiments or combination of embodiments thereof, as set forth above, and, optionally, another sweetener.

In certain particular embodiments, the comestible composition comprises sucrose and the MC1 compound. In some such embodiments, the introduction of the MC1 compound permits one to use less sucrose (such as more than 10% less, more than 20% less, more than 30% less, more than 40% less, more than 50% less, more than 60% less, or more than 70% less) and still achieve a level of sweetness characteristic of a comparable product that employs more sucrose. In some embodiments, the concentration of the MC1 compound is no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. The concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like. The sucrose can be introduced in any suitable form, such as natural syrups (cane syrup) and the like.

In certain particular embodiments, the comestible composition comprises fructose and the MC1 compound. In some such embodiments, the introduction of the MC1 compound permits one to use less fructose (such as more than 10% less, more than 20% less, more than 30% less, more than 40% less, more than 50% less, more than 60% less, or more than 70% less) and still achieve a level of sweetness characteristic of a comparable product that employs more fructose. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. In some embodiments, the comestible composition is a food product, such as any of those specifically listed below. In other embodiments, the comestible composition is a beverage product, such as a soda, and the like. The fructose can be supplied in any suitable form, such as natural syrups, high-fructose corn syrup, and the like.

In certain particular embodiments, the comestible composition comprises high-fructose corn syrup and the MC1 compound. In some such embodiments, the introduction of the MC1 compound permits one to use less high-fructose corn syrup (such as more than 10% less, more than 20% less, more than 30% less, more than 40% less, more than 50% less, more than 60% less, or more than 70% less) and still achieve a level of sweetness characteristic of a comparable product that employs more high-fructose corn syrup. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. In some embodiments, the comestible composition is a food product, such as any of those specifically listed below. In other embodiments, the comestible composition is a beverage product, such as a soda, and the like.

In certain particular embodiments, the comestible composition comprises glucose (for example, D-glucose, in either its alpha or beta forms, or a combination thereof) and the MC1 compound. In some such embodiments, the introduction of the MC1 compound permits one to use less glucose (such as more than 10% less, more than 20% less, more than 30% less, more than 40% less, more than 50% less, more than 60% less, or more than 70% less) and still achieve a level of sweetness characteristic of a comparable product that employs more glucose. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like. The glucose can be introduced in any suitable form, such as natural syrups and the like.

In certain particular embodiments, the comestible composition comprises sucralose and the MC1 compound. In some such embodiments, the introduction of the MC1 compound permits one to use less sucralose (such as more than 10% less, more than 20% less, more than 30% less, more than 40% less, more than 50% less, more than 60% less, or more than 70% less) and still achieve a level of sweetness characteristic of a comparable product that employs more sucralose. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like.

In certain particular embodiments, the comestible composition comprises rebaudiosides (such as rebaudioside A, rebaudioside D, rebaudioside E, rebaudioside M, or any combination thereof) and the MC1 compound. In some such embodiments, the introduction of the MC1 compound permits one to use less rebaudioside (such as more than 10% less, more than 20% less, more than 30% less, more than 40% less, more than 50% less, more than 60% less, or more than 70% less) and still achieve a level of sweetness characteristic of a comparable product that employs more rebaudioside. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like.

In certain particular embodiments, the comestible composition comprises acefulfame K and the MC1 compound. In some such embodiments, the introduction of the MC1 compound permits one to use less acesulfame K (such as more than 10% less, more than 20% less, more than 30% less, more than 40% less, more than 50% less, more than 60% less, or more than 70% less) and still achieve a level of sweetness characteristic of a comparable product that employs more acesulfame K. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like.

In certain particular embodiments, the comestible composition comprises allulose and the MC1 compound. In some such embodiments, the introduction of the MC1 compound permits one to use less allulose (such as more than 10% less, more than 20% less, more than 30% less, more than 40% less, more than 50% less, more than 60% less, or more than 70% less) and still achieve a level of sweetness characteristic of a comparable product that employs more allulose. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In some particular embodiments, the comestible composition is a protein bar or a meal replacement bar. In other embodiments, the comestible composition is a beverage product, such as a soda, and the like.

In certain particular embodiments, the comestible composition comprises erythritol and the MC1 compound. In some such embodiments, the introduction of the MC1 compound permits one to use less erythritol (such as more than 10% less, more than 20% less, more than 30% less, more than 40% less, more than 50% less, more than 60% less, or more than 70% less) and still achieve a level of sweetness characteristic of a comparable product that employs more erythritol. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like.

In certain particular embodiments, the comestible composition comprises aspartame and the MC1 compound. In some such embodiments, the introduction of the MC1 compound permits one to use less aspartame (such as more than 10% less, more than 20% less, more than 30% less, more than 40% less, more than 50% less, more than 60% less, or more than 70% less) and still achieve a level of sweetness characteristic of a comparable product that employs more aspartame. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like.

In certain particular embodiments, the comestible composition comprises cyclamate and the MC1 compound. In some such embodiments, the introduction of the MC1 compound permits one to use less cyclamate (such as more than 10% less, more than 20% less, more than 30% less, more than 40% less, more than 50% less, more than 60% less, or more than 70% less) and still achieve a level of sweetness characteristic of a comparable product that employs more cyclamate. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like.

In certain particular embodiments, the comestible composition comprises another mogroside (such as mogroside III, mogroside IV, mogroside V, siamenoside I, isomogroside V, mogroside IV_{E}, isomogroside IV, mogroside III_{E}, 11-oxomogroside V, the 1,6-α isomer of siamenoside I, and any combinations thereof) and the MC1 compound. In some such embodiments, the introduction of the MC1 compound permits one to use less of another mogroside (such as more than 10% less, more than 20% less, more than 30% less, more than 40% less, more than 50% less, more than 60% less, or more than 70% less) and still achieve a level of sweetness characteristic of a comparable product that employs more of the other mogroside. In some embodiments, the concentration of the MC1 compound is no more than 1000 ppm, or no more than 900 ppm, or no more than 800 ppm, or no more than 700 ppm, or no more than 600 ppm, or no more than 500 ppm, or no more than 400 ppm, or no more than 300 ppm, or no more than 200 ppm, or no more than 100 ppm, or no more than 50 ppm, or no more than 25 ppm, or no more than 10 ppm. In some further such embodiments, the concentration of the MC1 compound is at least 5 ppm. Such comestible compositions can be in any suitable form. In some embodiments, the comestible composition is a food product, such as any of those specifically listed below. In other embodiments, the comestible composition is a beverage product, such as a soda, and the like. Additional mogroside compounds that may be suitably used are described in U.S. Patent Application Publication No. 2017/0119032.

In certain embodiments of any aspects and embodiments set forth herein that refer to a comestible composition, the comestible composition is a non-naturally-occurring product, such as a composition specifically manufactured for the production of a flavored product, such as manufactured food or beverage product.

In general, compounds as disclosed and described herein, individually or in combination, can be provided in a composition, such as a comestible composition. In one embodiment, compounds as disclosed and described herein, individually or in combination, can impart a more sugar-like temporal profile or flavor profile to a sweetener composition by combining one or more of the compounds as disclosed and described herein with one or more sweeteners in the sweetener composition. In another embodiment, compounds as disclosed and described herein, individually or in combination, can increase or enhance the sweet taste of a composition by contacting the composition thereof with the compounds as disclosed and described herein to form a modified composition.

Thus, the compositions set forth in any of the foregoing aspects (including in any uses or methods), comprise the MC1 compound. In some embodiments, the composition further comprises a vehicle. In some embodiments, the vehicle is water. In some other embodiments, the vehicle is a bulking agent. In some other embodiments, the MC1 compound is present at a concentration at or above its sweetness recognition threshold.

In some embodiments, an additional sweetener is present. The additional sweetener can be present in any suitable concentration, depending on factors such as the sweetener's potency as a sweetener, its water solubility, and the like. For example, in some embodiments, the additional sweetener is present in an amount from 0.1% to 12% by weight. In some embodiments, the additional sweetener is present in an amount from 0.2% to 10% by weight. In some embodiments, the additional sweetener is present in an amount from 0.3% to 8% by weight. In some embodiments, the additional sweetener is present in an amount from 0.4% to 6% by weight. In some embodiments, the additional sweetener is present in an amount from 0.5% to 5% by weight. In some embodiments, the additional sweetener is present in an amount from 1% to 2% by weight. In some embodiments, the additional sweetener is present in an amount from 0.1% to 5% by weight. In some embodiments, the additional sweetener is present in an amount from 0.1% to 4% by weight. In some embodiments, the additional sweetener is present in an amount from 0.1% to 3% by weight. In some embodiments, the additional sweetener is present in an amount from 0.1% to 2% by weight. In some embodiments, the additional sweetener is present in an amount from 0.1% to 1% by weight. In some embodiments, the additional sweetener is present in an amount from 0.1% to 0.5% by weight. In some embodiments, the additional sweetener is present in an amount from 0.5% to 10% by weight. In some embodiments, the additional sweetener is present in an amount from 2% to 8% by weight. In some further embodiments of the embodiments set forth in this paragraph, the additional sweetener is sucrose, fructose, glucose, xylitol, erythritol, glucose, allulose, or combinations thereof.

In some other embodiments, the additional sweetener is present in an amount from 10 ppm to 1000 ppm. In some embodiments, the additional sweetener is present in an amount from 20 ppm to 800 ppm. In some embodiments, the additional sweetener is present in an amount from 30 ppm to 600 ppm. In some embodiments, the additional sweetener is present in an amount from 40 ppm to 500 ppm. In some embodiments, the additional sweetener is present in an amount from 50 ppm to 400 ppm. In some embodiments, the additional sweetener is present in an amount from 50 ppm to 300 ppm. In some embodiments, the additional sweetener is present in an amount from 50 ppm to 200 ppm. In some embodiments, the additional sweetener is present in an amount from 50 ppm to 150 ppm. In some further embodiments of the embodiments set forth in this paragraph, the additional sweetener is a steviol glycoside, a mogroside, a derivative of either of the foregoing, such as glycoside derivatives (e.g., glucosylates), or any combination thereof.

The compositions can include any suitable sweeteners or combination of sweeteners. In some embodiments, the sweetener is a common saccharide sweeteners, such as sucrose, fructose, glucose, and sweetener compositions comprising natural sugars, such as corn syrup (including high fructose corn syrup) or other syrups or sweetener concentrates derived from natural fruit and vegetable sources. In some embodiments, the sweetener is sucrose, fructose, or a combination thereof. In some embodiments, the sweetener is sucrose. In some other embodiments, the sweetener is selected from rare natural sugars including D-allose, D-psicose, L-ribose, D-tagatose, L-glucose, L-fucose, L-arbinose, D-turanose, and D-leucrose. In some embodiments, the sweetener is selected from semi-synthetic "sugar alcohol" sweeteners such as erythritol, isomalt, lactitol, mannitol, sorbitol, xylitol, maltodextrin, and the like. In some embodiments, the sweetener is selected from artificial sweeteners such as aspartame, saccharin, acesulfame-K, cyclamate, sucralose, and alitame. In some embodiments, the sweetener is selected from the group consisting of cyclamic acid, mogroside, tagatose, maltose, galactose, mannose, sucrose, fructose, lactose, allulose, neotame and other aspartame derivatives, glucose, D-tryptophan, glycine, maltitol, lactitol, isomalt, hydrogenated glucose syrup (HGS), hydrogenated starch hydrolyzate (HSH), stevioside, rebaudioside A, other sweet Stevia-based glycosides, chemically modified steviol glycosides (such as glucosylated steviol glycosides), mogrosides, chemically modified mogrosides (such as glucosylated mogrosides), carrelame and other guanidine-based sweeteners. In some embodiments, the additional sweetener is a combination of two or more of the sweeteners set forth in this paragraph. In some embodiments, the sweetener may combinations of two, three, four or five sweeteners as disclosed herein. In some embodiments, the additional sweetener is a sugar. In some embodiments, the additional sweetener is a combination of one or more sugars and other natural and artificial sweeteners. In some embodiments, the additional sweetener is a sugar. In some embodiments, the sugar is cane sugar. In some embodiments, the sugar is beet sugar. In some embodiments, the sugar may be sucrose, fructose, glucose or combinations thereof. In some embodiments, the sugar is sucrose. In some embodiments, the sugar is a combination of fructose and glucose.

In some embodiments, the additional sweeteners can also include, for example, sweetener compositions comprising one or more natural or synthetic carbohydrate, such as corn syrup, high fructose corn syrup, high maltose corn syrup, glucose syrup, sucralose syrup, hydrogenated glucose syrup (HGS), hydrogenated starch hydrolyzate (HSH), or other syrups or sweetener concentrates derived from natural fruit and vegetable sources, or semi-synthetic "sugar alcohol" sweeteners such as polyols. Non-limiting examples of polyols in some embodiments include erythritol, maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio-oligosaccharides, reduced maltose syrup, reduced glucose syrup, isomaltulose, maltodextrin, and the like, and sugar alcohols or any other carbohydrates or combinations thereof capable of being reduced which do not adversely affect taste.

The additional sweetener may be a natural or synthetic sweetener that includes, but is not limited to, agave inulin, agave nectar, agave syrup, amazake, brazzein, brown rice syrup, coconut crystals, coconut sugars, coconut syrup, date sugar, fructans (also referred to as inulin fiber, fructo-oligosaccharides, or oligo-fructose), green stevia powder, stevia rebaudiana, rebaudioside A, rebaudioside B, rebaudioside C, rebaudioside D, rebaudioside E, rebaudioside F, rebaudioside I, rebaudioside H, rebaudioside L, rebaudioside K, rebaudioside J, rebaudioside N, rebaudioside O, rebaudioside M and other sweet stevia-based glycosides, stevioside, stevioside extracts, honey, Jerusalem artichoke syrup, licorice root, luo han guo (fruit, powder, or extracts), lucuma (fruit, powder, or extracts), maple sap (including, for example, sap extracted from *Acer saccharum, Acer nigrum, Acer rubrum, Acer saccharinum, Acer platanoides, Acer negundo, Acer macrophyllum, Acer grandidentatum, Acer glabrum, Acer mono*), maple syrup, maple sugar, walnut sap (including, for example, sap extracted from *Juglans cinerea, Juglans nigra, Juglans ailatifolia, Juglans regia*)*,* birch sap (including, for example, sap extracted from *Betula papyrifera, Betula alleghaniensis, Betula lenta, Betula nigra, Betula populifolia, Betula pendula*), sycamore sap (such as, for example, sap extracted from *Platanus occidentalis*), ironwood sap (such as, for example, sap extracted from *Ostrya virginiana*), mascobado, molasses (such as, for example, blackstrap molasses), molasses sugar, monatin, monellin, cane sugar (also referred to as natural sugar, unrefined cane sugar, or sucrose), palm sugar, panocha, piloncillo, rapadura, raw sugar, rice syrup, sorghum, sorghum syrup, cassava syrup (also referred to as tapioca syrup), thaumatin, yacon root, malt syrup, barley malt syrup, barley malt powder, beet sugar, cane sugar, crystalline juice crystals, caramel, carbitol, carob syrup, castor sugar, hydrogenated starch hydrolates, hydrolyzed can juice, hydrolyzed starch, invert sugar, anethole, arabinogalactan, arrope, syrup, P-4000, acesulfame potassium (also referred to as acesulfame K or ace-K), alitame (also referred to as aclame), advantame, aspartame, baiyunoside, neotame, benzamide derivatives, bernadame, canderel, carrelame and other guanidine-based sweeteners, vegetable fiber, corn sugar, coupling sugars, curculin, cyclamates, cyclocarioside I, demerara, dextran, dextrin, diastatic malt, dulcin, sucrol, valzin, dulcoside A, dulcoside B, emulin, enoxolone, maltodextrin, saccharin, estragole, ethyl maltol, glucin, gluconic acid, glucono-lactone, glucosamine, glucoronic acid, glycerol, glycine, glycyphillin, glycyrrhizin, glycyrrhetic acid monoglucuronide, golden sugar, yellow sugar, golden syrup, granulated sugar, gynostemma, hernandulcin, isomerized liquid sugars, jallab, chicory root dietary fiber, kynurenine derivatives (including N'-formyl-kynurenine, N'-acetyl-kynurenine, 6-chloro-kynurenine), galactitol, litesse, ligicane, lycasin, lugduname, guanidine, falernum, mabinlin I, mabinlin II, maltol, maltisorb, maltodextrin, maltotriol, mannosamine, miraculin, mizuame, mogrosides (including, for example, mogroside IV, mogroside V, and neomogroside), mukurozioside, nano sugar, naringin dihydrochalcone, neohesperidine dihydrochalcone, nib sugar, nigero-oligosaccharide, norbu, orgeat syrup, osladin, pekmez, pentadin, periandrin I, perillaldehyde, perillartine, petphyllum, phenylalanine, phlomisoside I, phlorodizin, phyllodulcin, polyglycitol syrups, polypodoside A, pterocaryoside A, pterocaryoside B, rebiana, refiners syrup, rub syrup, rubusoside, selligueain A, shugr, siamenoside I, siraitia grosvenorii, soybean oligosaccharide, Splenda, SRI oxime V, steviol glycoside, steviolbioside, stevioside, strogins 1, 2, and 4, sucronic acid, sucrononate, sugar, suosan, phloridzin, superaspartame, tetrasaccharide, threitol, treacle, trilobtain, tryptophan and derivatives (6-trifluoromethyl-tryptophan, 6-chloro-D-tryptophan), vanilla sugar, volemitol, birch syrup, aspartame-acesulfame, assugrin, and combinations or blends of any two or more thereof.

In still other embodiments, the additional sweetener can be a chemically or enzymatically modified natural high potency sweetener. Modified natural high potency sweeteners include glycosylated natural high potency sweetener such as glucosyl-, galactosyl-, or fructosyl- derivatives containing 1-50 glycosidic residues. Glycosylated natural high potency sweeteners may be prepared by enzymatic transglycosylation reaction catalyzed by various enzymes possessing transglycosylating activity. In some embodiments, the modified sweetener can be substituted or unsubstituted.

Additional sweeteners also include combinations of any two or more of any of the aforementioned sweeteners. In some embodiments, the sweetener may comprise combinations of two, three, four or five sweeteners as disclosed herein. In some embodiments, the sweetener may be a sugar. In some embodiments, the sweetener may be a combination of one or more sugars and other natural and artificial sweeteners. In some embodiments, the sweetener is a caloric sweetener, such as sucrose, fructose, xylitol, erythritol, or combinations thereof. In some embodiments, the comestible compositions are free (or, in some embodiments) substantially free of stevia-derived sweeteners, such as steviol glycosides, glucosylated steviol glycosides, or rebaudiosides. For example, in some embodiments, the comestible compositions are either free of stevia-derived sweeteners or comprise stevia-derived sweeteners in a concentration of no more than 1000 ppm, or no more than 500 ppm, or no more than 200 ppm, or no more than 100 ppm, or no more than 50 ppm, or no more than 20 ppm, or no more than 10 ppm, or no more than 5 ppm, or no more than 3 ppm, or no more than 1 ppm.

The MC1 compound is present in the comestible compositions at a concentration ranging from 5 to 1000 ppm. In some embodiments, the MC1 compound is present in an amount sufficient to enhance the taste (e.g., enhance the sweetness, reduce the sourness, or reduce the bitterness) of the compositions. Thus, in some embodiments, the comestible composition comprises the MC1 compound in a concentration no greater than 900 ppm, or no greater than 800 ppm, or no greater than 700 ppm, or no greater than 600 ppm, or no greater than 500 ppm, or no greater than 450 ppm, or no greater than 400 ppm, or no greater than 350 ppm, or no greater than 300 ppm, or no greater than 250 ppm, or no greater than 200 ppm, or no greater than 150 ppm, or no greater than 100 ppm, or no greater than 50 ppm, or no greater than 40 ppm, or no greater than 30 ppm, or no greater than 20 ppm. The mogroside compound is present in a minimum amount of 5 ppm. It is disclosed in context of the use of claim 1 that, the comestible composition may comprises the MC1 compound in a concentration ranging from 1 ppm to 1000 ppm, or from 1 ppm to 900 ppm, or from 1 ppm to 800 ppm, or from 1 ppm to 700 ppm, or from 1 ppm to 600 ppm, or from 1 ppm to 500 ppm, or from 1 ppm to 450 ppm, or from 1 ppm to 400 ppm, or from 1 ppm to 350 ppm, or from 1 ppm to 300 ppm, or from 1 ppm to 250 ppm, or from 1 ppm to 200 ppm, or from 1 ppm to 150 ppm, or from 1 ppm to 100 ppm, or from 1 ppm to 50 ppm, or from 1 ppm to 40 ppm, or from 1 ppm to 30 ppm, or from 1 ppm to 20 ppm. In some embodiments, the comestible composition comprises the mogroside compound in a concentration fromor from 5 ppm to 900 ppm, or from 5 ppm to 800 ppm, or from 5 ppm to 700 ppm, or from 5 ppm to 600 ppm, or from 5 ppm to 500 ppm, or from 5 ppm to 450 ppm, or from 5 ppm to 400 ppm, or from 5 ppm to 350 ppm, or from 5 ppm to 300 ppm, or from 5 ppm to 250 ppm, or from 5 ppm to 200 ppm, or from 5 ppm to 150 ppm, or from 5 ppm to 100 ppm, or from 5 ppm to 50 ppm, or from 5 ppm to 40 ppm, or from 5 ppm to 30 ppm, or from 5 ppm to 20 ppm. In embodiments where a sweetener, such as sucrose or fructose, are present, the weight-to-weight ratio of sweetener to the MC1 compound in the comestible composition ranges from 1000:1 to 50000:1, or from 1000:1 to 10000:1, or from 2000:1 to 8000:1.

The comestible compositions or sweetener concentrates comprising the MC1 compound can, in certain embodiments, comprise any additional ingredients or combination of ingredients as are commonly used in food and beverage products, including, but not limited to:
acids, including, for example citric acid, phosphoric acid, ascorbic acid, sodium acid sulfate, lactic acid, or tartaric acid;
bitter ingredients, including, for example caffeine, quinine, green tea, catechins, polyphenols, green robusta coffee extract, green coffee extract, potassium chloride, menthol, or proteins (such as proteins and protein isolates derived from plants, algae, or fungi);
coloring agents, including, for example caramel color, Red #40, Yellow #5, Yellow #6, Blue #1, Red #3, purple carrot, black carrot juice, purple sweet potato, vegetable juice, fruit juice, beta carotene, turmeric curcumin, or titanium dioxide;
preservatives, including, for example sodium benzoate, potassium benzoate, potassium sorbate, sodium metabisulfate, sorbic acid, or benzoic acid;
antioxidants including, for example ascorbic acid, calcium disodium EDTA, alpha tocopherols, mixed tocopherols, rosemary extract, grape seed extract, resveratrol, or sodium hexametaphosphate;
vitamins or functional ingredients including, for example resveratrol, Co-Q10, omega 3 fatty acids, theanine, choline chloride (citocoline), fibersol, inulin (chicory root), taurine, panax ginseng extract, guanana extract, ginger extract, L-phenylalanine, L-carnitine, L-tartrate, D-glucoronolactone, inositol, bioflavonoids, Echinacea, ginko biloba, yerba mate, flax seed oil, garcinia cambogia rind extract, white tea extract, ribose, milk thistle extract, grape seed extract, pyrodixine HCl (vitamin B6), cyanoobalamin (vitamin B12), niacinamide (vitamin B3), biotin, calcium lactate, calcium pantothenate (pantothenic acid), calcium phosphate, calcium carbonate, chromium chloride, chromium polynicotinate, cupric sulfate, folic acid, ferric pyrophosphate, iron, magnesium lactate, magnesium carbonate, magnesium sulfate, monopotassium phosphate, monosodium phosphate, phosphorus, potassium iodide, potassium phosphate, riboflavin, sodium sulfate, sodium gluconate, sodium polyphosphate, sodium bicarbonate, thiamine mononitrate, vitamin D3, vitamin A palmitate, zinc gluconate, zinc lactate, or zinc sulphate;
clouding agents, including, for example ester gun, brominated vegetable oil (BVO), or sucrose acetate isobutyrate (SAIB);
buffers, including, for example sodium citrate, potassium citrate, or salt;
flavors, including, for example propylene glycol, ethyl alcohol, glycerine, gum Arabic (gum acacia), maltodextrin, modified corn starch, dextrose, natural flavor, natural flavor with other natural flavors (natural flavor WONF), natural and artificial flavors, artificial flavor, silicon dioxide, magnesium carbonate, or tricalcium phosphate; or
starches and stabilizers, including, for example pectin, xanthan gum, carboxylmethylcellulose (CMC), polysorbate 60, polysorbate 80, medium chain triglycerides, cellulose gel, cellulose gum, sodium caseinate, modified food starch, gum Arabic (gum acacia), inulin, or carrageenan.

The comestible compositions or sweetener concentrates comprising the MC1 compound can have any suitable pH. In some embodiments, the mogroside compounds enhance the sweetness of a sweetener under a broad range of pH, e.g., from lower pH to neutral pH. The lower and neutral pH includes, but is not limited to, a pH from 1.5 to 9.0, or from 2.5 to 8.5; from 3.0 to 8.0; from 3.5 to 7.5; and from 4.0 to 7. In certain embodiments, te MC1 compound can enhance the perceived sweetness of a fixed concentration of a sweetener in taste tests at a compound concentration of 50 µM, 40 µM, 30 µM, 20 µM, or 10 µM at both low to neutral pH value. In certain embodiments, the enhancement factor of the compounds as disclosed and described herein, individually or in combination, at the lower pH is substantially similar to the enhancement factor of the compounds at neutral pH. Such consistent sweet enhancing property under a broad range of pH allow a broad use in a wide variety of foods and beverages of the compounds as disclosed and described herein, individually or in combination.

The comestible compositions set forth according to any of the foregoing embodiments, also include, in certain embodiments, one or more additional flavor-modifying compounds, such as compounds that enhance sweetness (e.g., hesperetin, naringenin, glucosylated steviol glycosides, etc.), compounds that block bitterness, compounds that enhance umami, compounds that reduce sourness or licorice taste, compounds that enhance saltiness, compounds that enhance a cooling effect, or any combinations of the foregoing.

Thus, in some embodiments, comestible compositions disclosed herein comprise the MC1 compound are combined with one or more other sweetness enhancing compounds. Such sweetness enhancing compounds include, but are not limited to, naturally derived compounds, such as hesperitin, naringenin, glucosylated steviol glycosides, or synthetic compounds, such as any compounds set forth in U.S. Patent Nos. 8,541,421; 8,815,956; 9,834,544; 8,592,592; 8,877,922; 9,000,054; and 9,000,051, as well as U.S. Patent Application Publication No. 2017/0119032. The MC1 compound may be used in combination with such other sweetness enhancers in any suitable ratio (w/w) ranging from 1:1000 to 1000:1, or from 1:100 to 100:1, or from, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1. In some embodiments of any of the preceding embodiments, the MC1 compound is combined with glucosylated steviol glycosides in any of the above ratios. As used herein, the term "glucosylated steviol glycoside" refers to the product of enzymatically glucosylating natural steviol glycoside compounds. The glucosylation generally occurs through a glycosidic bond, such as an α-1,2 bond, an α-1,4 bond, an α-1.6 bond, a β-1,2 bond, a β-1,4 bond, a β-1,6 bond, and so forth. In some embodiments of any of the preceding embodiments, the MC1 compound is combined with 3-((4-amino-2,2-dioxo-1H-benzo[c][1,2,6]thiadiazin-5-yl)oxy)-2,2-dimethyl-N-propyl-propanamide, in any of the above ratios.

In some further embodiments, comestible compositions disclosed herein comprise the MC1 compound are combined with one or more umami enhancing compounds. Such umami enhancing compounds include, but are not limited to, naturally derived compounds, such as ericamide, or synthetic compounds, such as any compounds set forth in U.S. Patent Nos. 8,735,081; 8,124,121; and 8,968,708. The MC1 compound may be used in combination with such umami enhancers in any suitable ratio (w/w) ranging from 1:1000 to 1000:1, or from 1:100 to 100:1, or from, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1.

In some further embodiments, comestible compositions disclosed herein comprise the MC1 compound are combined with one or more cooling enhancing compounds. Such cooling enhancing compounds include, but are not limited to, naturally derived compounds, such as menthol or analogs thereof, or synthetic compounds, such as any compounds set forth in U.S. Patent Nos. 9,394,287 and 10,421,727. The MC1 compound may be used in combination with such umami enhancers in any suitable ratio (w/w) ranging from 1:1000 to 1000:1, or from 1:100 to 100:1, or from, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1.

In some further embodiments, comestible compositions disclosed herein comprise the MC1 compound are combined with one or more bitterness blocking compounds. Such bitterness blocking compounds include, but are not limited to, naturally derived compounds, such as menthol or analogs thereof, or synthetic compounds, such as any compounds set forth in U.S. Patent Nos. 8,076,491; 8,445,692; and 9,247,759. The MC1 compound may be used in combination with such bitterness blockers in any suitable ratio (w/w) ranging from 1:1000 to 1000:1, or from 1:100 to 100:1, or from, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1.

In some further embodiments, comestible compositions disclosed herein comprise the MC1 compound are combined with one or more sour taste modulating compounds. The MC1 compound may be used in combination with such sour taste modulating compounds in any suitable ratio (w/w) ranging from 1:1000 to 1000:1, or from 1:100 to 100:1, or from, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1.

In some further embodiments, comestible compositions disclosed herein comprise the MC1 compound are combined with one or more mouthfeel modifying compounds. Such mouthfeel modifying compounds include, but are not limited to, tannins, cellulosic materials, bamboo powder, and the like. The MC1 compound may be used in combination with such mouthfeel enhancers in any suitable ratio (w/w) ranging from 1:1000 to 1000:1, or from 1:100 to 100:1, or fro, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1.

In some further embodiments, comestible compositions disclosed herein comprise the MC1 compound are combined with one or more flavor masking compounds. Such flavor masking compounds include, but are not limited to, cellulosic materials, materials extracted from fungus, materials extracted from plants, citric acid, carbonic acid (or carbonates), and the like. The MC1 compound may be used in combination with such mouthfeel enhancers in any suitable ratio (w/w) ranging from 1:1000 to 1000:1, or from 1:100 to 100:1, or from, 1:50 to 50:1, or from 1:25 to 25:1, or from 1:10 to 10:1, such as 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, or 25:1.

In some aspects related to the preceding aspects and embodiments, the disclosure provides uses of the MC1 compound to enhance the flavor of a flavored composition, such as a flavored article. Such flavored compositions can use any suitable flavors, such as any of the flavors set forth above.

### Flavored Products and Concentrates

In certain aspects, the disclosure provides flavored products comprising any compositions of the preceding aspects. In some embodiment, the flavored products are beverage products, such as soda, flavored water, tea, and the like. In some other embodiments, the flavored products are food products, such as yogurt.

In embodiments where the flavored product is a beverage, the beverage may be selected from the group consisting of enhanced sparkling beverages, colas, lemon-lime flavored sparkling beverages, orange flavored sparkling beverages, grape flavored sparkling beverages, strawberry flavored sparkling beverages, pineapple flavored sparkling beverages, ginger-ales, root beers, fruit juices, fruit-flavored juices, juice drinks, nectars, vegetable juices, vegetable-flavored juices, sports drinks, energy drinks, enhanced water drinks, enhanced water with vitamins, near water drinks, coconut waters, tea type drinks, coffees, cocoa drinks, beverages containing milk components, beverages containing cereal extracts and smoothies. In some embodiments, the beverage may be a soft drink.

In certain embodiments of any aspects and embodiments set forth herein that refer to an flavored product, the flavored product is a non-naturally-occurring product, such as a packaged food or beverage product.

Further non-limiting examples of food and beverage products or formulations include sweet coatings, frostings, or glazes for such products or any entity included in the Soup category, the Dried Processed Food category, the Beverage category, the Ready Meal category, the Canned or Preserved Food category, the Frozen Processed Food category, the Chilled Processed Food category, the Snack Food category, the Baked Goods category, the Confectionery category, the Dairy Product category, the Ice Cream category, the Meal Replacement category, the Pasta and Noodle category, and the Sauces, Dressings, Condiments category, the Baby Food category, and/or the Spreads category.

In general, the Soup category refers to canned/preserved, dehydrated, instant, chilled, UHT and frozen soup. For the purpose of this definition soup(s) means a food prepared from meat, poultry, fish, vegetables, grains, fruit and other ingredients, cooked in a liquid which may include visible pieces of some or all of these ingredients. It may be clear (as a broth) or thick (as a chowder), smooth, pureed or chunky, ready-to-serve, semi-condensed or condensed and may be served hot or cold, as a first course or as the main course of a meal or as a between meal snack (sipped like a beverage). Soup may be used as an ingredient for preparing other meal components and may range from broths (consommé) to sauces (cream or cheese-based soups).

The Dehydrated and Culinary Food Category usually means: (i) Cooking aid products such as: powders, granules, pastes, concentrated liquid products, including concentrated bouillon, bouillon and bouillon like products in pressed cubes, tablets or powder or granulated form, which are sold separately as a finished product or as an ingredient within a product, sauces and recipe mixes (regardless of technology); (ii) Meal solutions products such as: dehydrated and freeze dried soups, including dehydrated soup mixes, dehydrated instant soups, dehydrated ready-to-cook soups, dehydrated or ambient preparations of ready-made dishes, meals and single serve entrees including pasta, potato and rice dishes; and (iii) Meal embellishment products such as: condiments, marinades, salad dressings, salad toppings, dips, breading, batter mixes, shelf stable spreads, barbecue sauces, liquid recipe mixes, concentrates, sauces or sauce mixes, including recipe mixes for salad, sold as a finished product or as an ingredient within a product, whether dehydrated, liquid or frozen.

The Beverage category usually means beverages, beverage mixes and concentrates, including but not limited to, carbonated and non-carbonated beverages, alcoholic and non-alcoholic beverages, ready to drink beverages, liquid concentrate formulations for preparing beverages such as sodas, and dry powdered beverage precursor mixes. The Beverage category also includes the alcoholic drinks, the soft drinks, sports drinks, isotonic beverages, and hot drinks. The alcoholic drinks include, but are not limited to beer, cider/perry, FABs, wine, and spirits. The soft drinks include, but are not limited to carbonates, such as colas and non-cola carbonates; fruit juice, such as juice, nectars, juice drinks and fruit flavored drinks; bottled water, which includes sparkling water, spring water and purified/table water; functional drinks, which can be carbonated or still and include sport, energy or elixir drinks; concentrates, such as liquid and powder concentrates in ready to drink measure. The drinks, either hot or cold, include, but are not limited to coffee or ice coffee, such as fresh, instant, and combined coffee; tea or ice tea, such as black, green, white, oolong, and flavored tea; and other drinks including flavor-, malt- or plant-based powders, granules, blocks or tablets mixed with milk or water.

The Snack Food category generally refers to any food that can be a light informal meal including, but not limited to Sweet and savory snacks and snack bars. Examples of snack food include, but are not limited to fruit snacks, chips/crisps, extruded snacks, tortilla/corn chips, popcorn, pretzels, nuts and other sweet and savory snacks. Examples of snack bars include, but are not limited to granola/muesli bars, breakfast bars, energy bars, fruit bars and other snack bars.

The Baked Goods category generally refers to any edible product the process of preparing which involves exposure to heat or excessive sunlight. Examples of baked goods include, but are not limited to bread, buns, cookies, muffins, cereal, toaster pastries, pastries, waffles, tortillas, biscuits, pies, bagels, tarts, quiches, cake, any baked foods, and any combination thereof.

The Ice Cream category generally refers to frozen dessert containing cream and sugar and flavoring. Examples of ice cream include, but are not limited to: impulse ice cream; take-home ice cream; frozen yoghurt and artisanal ice cream; soy, oat, bean (e.g., red bean and mung bean), and rice-based ice creams.

The Confectionery category generally refers to edible product that is sweet to the taste. Examples of confectionery include, but are not limited to candies, gelatins, chocolate confectionery, sugar confectionery, gum, and the likes and any combination products.

The Meal Replacement category generally refers to any food intended to replace the normal meals, particularly for people having health or fitness concerns. Examples of meal replacement include, but are not limited to slimming products and convalescence products.

The Ready Meal category generally refers to any food that can be served as meal without extensive preparation or processing. The ready meal includes products that have had recipe "skills" added to them by the manufacturer, resulting in a high degree of readiness, completion and convenience. Examples of ready meal include, but are not limited to canned/preserved, frozen, dried, chilled ready meals; dinner mixes; frozen pizza; chilled pizza; and prepared salads.

The Pasta and Noodle category includes any pastas and/or noodles including, but not limited to canned, dried and chilled/fresh pasta; and plain, instant, chilled, frozen and snack noodles.

The Canned/Preserved Food category includes, but is not limited to canned/preserved meat and meat products, fish/seafood, vegetables, tomatoes, beans, fruit, ready meals, soup, pasta, and other canned/preserved foods.

The Frozen Processed Food category includes, but is not limited to frozen processed red meat, processed poultry, processed fish/seafood, processed vegetables, meat substitutes, processed potatoes, bakery products, desserts, ready meals, pizza, soup, noodles, and other frozen food.

The Dried Processed Food category includes, but is not limited to rice, dessert mixes, dried ready meals, dehydrated soup, instant soup, dried pasta, plain noodles, and instant noodles. The Chill Processed Food category includes, but is not limited to chilled processed meats, processed fish/seafood products, lunch kits, fresh cut fruits, ready meals, pizza, prepared salads, soup, fresh pasta and noodles.

The Sauces, Dressings and Condiments category includes, but is not limited to tomato pastes and purees, bouillon/stock cubes, herbs and spices, monosodium glutamate (MSG), table sauces, soy based sauces, pasta sauces, wet/cooking sauces, dry sauces/powder mixes, ketchup, mayonnaise, mustard, salad dressings, vinaigrettes, dips, pickled products, and other sauces, dressings and condiments.

The Baby Food category includes, but is not limited to milk- or soybean-based formula; and prepared, dried and other baby food.

The Spreads category includes, but is not limited to jams and preserves, honey, chocolate spreads, nut based spreads, and yeast based spreads.

The Dairy Product category generally refers to edible product produced from mammal's milk. Examples of dairy product include, but are not limited to drinking milk products, cheese, yoghurt and sour milk drinks, and other dairy products.

Additional examples for flavored products, particularly food and beverage products or formulations, are provided as follows. Exemplary comestible compositions include one or more confectioneries, chocolate confectionery, tablets, countlines, bagged selflines/softlines, boxed assortments, standard boxed assortments, twist wrapped miniatures, seasonal chocolate, chocolate with toys, alfajores, other chocolate confectionery, mints, standard mints, power mints, boiled sweets, pastilles, gums, jellies and chews, toffees, caramels and nougat, medicated confectionery, lollipops, liquorice, other sugar confectionery, bread, packaged/industrial bread, unpackaged/artisanal bread, pastries, cakes, packaged/industrial cakes, unpackaged/artisanal cakes, cookies, chocolate coated biscuits, sandwich biscuits, filled biscuits, savory biscuits and crackers, bread substitutes, breakfast cereals, rte cereals, family breakfast cereals, flakes, muesli, other cereals, children's breakfast cereals, hot cereals, ice cream, impulse ice cream, single portion dairy ice cream, single portion water ice cream, multi-pack dairy ice cream, multi-pack water ice cream, take-home ice cream, take-home dairy ice cream, ice cream desserts, bulk ice cream, take-home water ice cream, frozen yoghurt, artisanal ice cream, dairy products, milk, fresh/pasteurized milk, full fat fresh/pasteurized milk, semi skimmed fresh/pasteurized milk, long-life/uht milk, full fat long life/uht milk, semi skimmed long life/uht milk, fat-free long life/uht milk, goat milk, condensed/evaporated milk, plain condensed/evaporated milk, flavored, functional and other condensed milk, flavored milk drinks, dairy only flavored milk drinks, flavored milk drinks with fruit juice, soy milk, sour milk drinks, fermented dairy drinks, coffee whiteners, powder milk, flavored powder milk drinks, cream, cheese, processed cheese, spreadable processed cheese, unspreadable processed cheese, unprocessed cheese, spreadable unprocessed cheese, hard cheese, packaged hard cheese, unpackaged hard cheese, yoghurt, plain/natural yoghurt, flavored yoghurt, fruited yoghurt, probiotic yoghurt, drinking yoghurt, regular drinking yoghurt, probiotic drinking yoghurt, chilled and shelf-stable desserts, dairy-based desserts, soy-based desserts, chilled snacks, fromage frais and quark, plain fromage frais and quark, flavored fromage frais and quark, savory fromage frais and quark, sweet and savory snacks, fruit snacks, chips/crisps, extruded snacks, tortilla/corn chips, popcorn, pretzels, nuts, other sweet and savory snacks, snack bars, granola bars, breakfast bars, energy bars, fruit bars, other snack bars, meal replacement products, slimming products, convalescence drinks, ready meals, canned ready meals, frozen ready meals, dried ready meals, chilled ready meals, dinner mixes, frozen pizza, chilled pizza, soup, canned soup, dehydrated soup, instant soup, chilled soup, hot soup, frozen soup, pasta, canned pasta, dried pasta, chilled/fresh pasta, noodles, plain noodles, instant noodles, cups/bowl instant noodles, pouch instant noodles, chilled noodles, snack noodles, canned food, canned meat and meat products, canned fish/seafood, canned vegetables, canned tomatoes, canned beans, canned fruit, canned ready meals, canned soup, canned pasta, other canned foods, frozen food, frozen processed red meat, frozen processed poultry, frozen processed fish/seafood, frozen processed vegetables, frozen meat substitutes, frozen potatoes, oven baked potato chips, other oven baked potato products, non-oven frozen potatoes, frozen bakery products, frozen desserts, frozen ready meals, frozen pizza, frozen soup, frozen noodles, other frozen food, dried food, dessert mixes, dried ready meals, dehydrated soup, instant soup, dried pasta, plain noodles, instant noodles, cups/bowl instant noodles, pouch instant noodles, chilled food, chilled processed meats, chilled fish/seafood products, chilled processed fish, chilled coated fish, chilled smoked fish, chilled lunch kit, chilled ready meals, chilled pizza, chilled soup, chilled/fresh pasta, chilled noodles, oils and fats, olive oil, vegetable and seed oil, cooking fats, butter, margarine, spreadable oils and fats, functional spreadable oils and fats, sauces, dressings and condiments, tomato pastes and purees, bouillon/stock cubes, stock cubes, gravy granules, liquid stocks and fonds, herbs and spices, fermented sauces, soy based sauces, pasta sauces, wet sauces, dry sauces/powder mixes, ketchup, mayonnaise, regular mayonnaise, mustard, salad dressings, regular salad dressings, low fat salad dressings, vinaigrettes, dips, pickled products, other sauces, dressings and condiments, baby food, milk formula, standard milk formula, follow-on milk formula, toddler milk formula, hypoallergenic milk formula, prepared baby food, dried baby food, other baby food, spreads, jams and preserves, honey, chocolate spreads, nut-based spreads, and yeast-based spreads. Exemplary comestible compositions also include confectioneries, bakery products, ice creams, dairy products, sweet and savory snacks, snack bars, meal replacement products, ready meals, soups, pastas, noodles, canned foods, frozen foods, dried foods, chilled foods, oils and fats, baby foods, or spreads or a mixture thereof. Exemplary comestible compositions also include breakfast cereals, sweet beverages or solid or liquid concentrate compositions for preparing beverages, ideally so as to enable the reduction in concentration of previously known saccharide sweeteners, or artificial sweeteners.

Some embodiments provide a chewable composition that may or may not be intended to be swallowed. In some embodiments, the chewable composition may be gum, chewing gum, sugarized gum, sugar-free gum, functional gum, bubble gum including compounds as disclosed and described herein, individually or in combination.

In some embodiments, the MC1 compound modulates the sweet taste or other taste properties of other natural or synthetic sweet tastants, and comestible compositions made therefrom. In one embodiment, the MC1 compound may be used or provided in its ligand enhancing concentration(s). For example, in some such embodiments, the MC1 compound is present in the comestible composition at a concentration ranging from 0.001 ppm to 100 ppm, or narrower alternative ranges from 0.1 ppm to 50 ppm, from 0.01 ppm to 40 ppm, from 0.05 ppm to 30 ppm, from 0.01 ppm to 25 ppm, or from 0.1 ppm to 30 ppm, or from 0.1 ppm to 25 ppm, or from 1 ppm to 30 ppm, or from 1 ppm to 25 ppm.

In some embodiments, the MC1 compound is provided in a flavoring concentrate formulation, e.g., suitable for subsequent processing to produce a ready-to-use (i.e., ready-to-serve) product. By "a flavoring concentrate formulation", it is meant a formulation which should be reconstituted with one or more diluting medium to become a ready-to-use composition. The term "ready-to-use composition" is used herein interchangeably with "comestible composition", which denotes any substance that, either alone or together with another substance, can be taken by mouth whether intended for consumption or not. In one embodiment, the ready-to-use composition includes a composition that can be directly consumed by a human or animal. The flavoring concentrate formulation is typically used by mixing with or diluted by one or more diluting medium, e.g., any consumable or comestible ingredient or product, to impart or modify one or more flavors to the diluting medium. Such a use process is often referred to as reconstitution. The reconstitution can be conducted in a household setting or an industrial setting. For example, a frozen fruit juice concentrate can be reconstituted with water or other aqueous medium by a consumer in a kitchen to obtain the ready-to-use fruit juice beverage. In another example, a soft drink syrup concentrate can be reconstituted with water or other aqueous medium by a manufacturer in large industrial scales to produce the ready-to-use soft drinks. Since the flavoring concentrate formulation has the flavoring agent or flavor modifying agent in a concentration higher than the ready-to-use composition, the flavoring concentrate formulation is typically not suitable for being consumed directly without reconstitution. There are many benefits of using and producing a flavoring concentrate formulation. For example, one benefit is the reduction in weight and volume for transportation as the flavoring concentrate formulation can be reconstituted at the time of usage by the addition of suitable solvent, solid or liquid.

In certain embodiments of any aspects and embodiments set forth herein that refer to a sweetening or flavoring concentrate, the sweetening or flavoring concentrate is a non-naturally-occurring product, such as a composition specifically manufactured for the production of a flavored product, such as food or beverage product.

In some embodiments, the flavoring concentrate formulation comprises: the MC1 compound; a carrier; and, optionally, at least one adjuvant. The term "carrier" denotes a usually inactive accessory substance, such as solvents, binders, bulking agents, or other inert medium, which is used in combination with the present compound and one or more optional adjuvants to form the formulation. For example, water or starch can be a carrier for a flavoring concentrate formulation. In some embodiments, the carrier is the same as the diluting medium for reconstituting the flavoring concentrate formulation; and in other embodiments, the carrier is different from the diluting medium. The term "carrier" as used herein includes, but is not limited to, comestibly acceptable carrier.

The term "adjuvant" denotes an additive which supplements, stabilizes, maintains, or enhances the intended function or effectiveness of the active ingredient, such as the compound of the present invention. In one embodiment, the at least one adjuvant comprises one or more flavoring agents. The flavoring agent may be of any flavor known to one skilled in the art or consumers, such as the flavor of chocolate, coffee, tea, mocha, French vanilla, peanut butter, chai, or combinations thereof. In another embodiment, the at least one adjuvant comprises one or more sweeteners. The one or more sweeteners can be any of the sweeteners described in this application. In another embodiment, the at least one adjuvant comprises one or more ingredients selected from the group consisting of a emulsifier, a stabilizer, an antimicrobial preservative, an antioxidant, vitamins, minerals, fats, starches, protein concentrates and isolates, salts, and combinations thereof. Examples of emulsifiers, stabilizers, antimicrobial preservatives, antioxidants, vitamins, minerals, fats, starches, protein concentrates and isolates, and salts are described in U.S. Pat. No. 6,468,576.

In some embodiments, the present flavoring concentrate formulation can be in a form selected from the group consisting of liquid including solution and suspension, solid, foamy material, paste, gel, cream, and a combination thereof, such as a liquid containing certain amount of solid contents. In one embodiment, the flavoring concentrate formulation is in form of a liquid including aqueous-based and nonaqueous-based. In some embodiments, the present flavoring concentrate formulation can be carbonated or non-carbonated.

The flavoring concentrate formulation may further comprise a freezing point depressant, nucleating agent, or both as the at least one adjuvant. The freezing point depressant is an ingestibly acceptable compound or agent which can depress the freezing point of a liquid or solvent to which the compound or agent is added. That is, a liquid or solution containing the freezing point depressant has a lower freezing point than the liquid or solvent without the freezing point depressant. In addition to depress the onset freezing point, the freezing point depressant may also lower the water activity of the flavoring concentrate formulation. The examples of the freezing point depressant include, but are not limited to, carbohydrates, oils, ethyl alcohol, polyol, e.g., glycerol, and combinations thereof. The nucleating agent denotes an ingestibly acceptable compound or agent which is able to facilitate nucleation. The presence of nucleating agent in the flavoring concentrate formulation can improve the mouthfeel of the frozen Blushes of a frozen slush and to help maintain the physical properties and performance of the slush at freezing temperatures by increasing the number of desirable ice crystallization centers. Examples of nucleating agents include, but are not limited to, calcium silicate, calcium carbonate, titanium dioxide, and combinations thereof.

In some embodiments, the flavoring concentrate formulation is formulated to have a low water activity for extended shelf life. Water activity is the ratio of the vapor pressure of water in a formulation to the vapor pressure of pure water at the same temperature. In one embodiment, the flavoring concentrate formulation has a water activity of less than about 0.85. In another embodiment, the flavoring concentrate formulation has a water activity of less than about 0.80. In another embodiment, the flavoring concentrate formulation has a water activity of less than about 0.75.

In some embodiments, the flavoring concentrate formulation has the present compound in a concentration that is at least 2 times of the concentration of the compound in a ready-to-use composition. In one embodiment, the flavoring concentrate formulation has the present compound in a concentration that is at least 5 times of the concentration of the compound in a ready-to-use composition. In one embodiment, the flavoring concentrate formulation has the present compound in a concentration that is at least 10 times of the concentration of the compound in a ready-to-use composition. In one embodiment, the flavoring concentrate formulation has the present compound in a concentration that is at least 15 times of the concentration of the compound in a ready-to-use composition. In one embodiment, the flavoring concentrate formulation has the present compound in a concentration that is at least 20 times of the concentration of the compound in a ready-to-use composition. In one embodiment, the flavoring concentrate formulation has the present compound in a concentration that is at least 30 times of the concentration of the compound in a ready-to-use composition. In one embodiment, the flavoring concentrate formulation has the present compound in a concentration that is at least 40 times of the concentration of the compound in a ready-to-use composition. In one embodiment, the flavoring concentrate formulation has the present compound in a concentration that is at least 50 times of the concentration of the compound in a ready-to-use composition. In one embodiment, the flavoring concentrate formulation has the present compound in a concentration that is at least 60 times of the concentration of the compound in a ready-to-use composition. In one embodiment, the flavoring concentrate formulation has the present compound in a concentration that is up to 100 times of the concentration of the compound in a ready-to-use composition.

The sweetening or flavoring concentrates set forth according to any of the foregoing embodiments, also include, in certain embodiments, one or more additional mogroside compounds, such as compounds that enhance sweetness (e.g., hesperetin, naringenin, glucosylated steviol glycosides, etc.), compounds that block bitterness (e.g., eriodictyol, homoeriodictyol, sterubin, and salts or glycoside derivatives thereof, as well as vanillyl lignans, e.g., matairesinol and other compounds set forth in PCT Publication No. WO 2012/146584), compounds that enhance umami (e.g., rubemamine, rubescenamine, (E)-3-(3,4-dimethoxyphenyl)-N-(4-methoxyphenethyl)acrylamide, and the like), compounds that reduce sourness and/or licorice taste, compounds that enhance saltiness, compounds that enhance a cooling effect, or any combinations of the foregoing.

In some embodiments, the comestible compositions comprising the MC1 compound also include one or more vanilla flavor compounds, such as 4-hydroxy-3-methoxybenzaldehyde or 2-hydroxy-4-methoxybenzaldehyde. Such comestible compositions can suitably be used in a variety of different flavored products, such as coffee-based drinks, protein mixes, meal-replacement drinks, ice cream, and the like.

### Tabletop Compositions

In some further aspects, the disclosure provides a tabletop sweetener composition comprising: (a) at least one sweetener composition according to any of the preceding aspects and embodiments thereof (namely, compositions comprising a sweetener and the MC1 compound); and (b) at least one bulking agent.

The tabletop sweetener composition may take any suitable form including, but not limited to, an amorphous solid, a crystal, a powder, a tablet, a liquid, a cube, a glace or coating, a granulated product, an encapsulated form abound to or coated on to carriers/particles, wet or dried, or combinations thereof.

The tabletop sweetener composition may contain further additives known to those skilled in the art. These additives include but are not limited to bubble forming agents, bulking agents, carriers, fibers, sugar alcohols, oligosaccharides, sugars, high intensity sweeteners, nutritive sweeteners, flavorings, flavor enhancers, flavor stabilizers, acidulants, anti-caking and free-flow agents. Such additives are for example described by H. Mitchell (H. Mitchell, SWEETENERS AND SUGAR ALTERNATIVES IN FOOD TECHNOLOGY, Blackwell Publishing Ltd, 2006). As used herein, the term "flavorings" may include those flavors known to the skilled person, such as natural and artificial flavors. These flavorings may be chosen from synthetic flavor oils and flavoring aromatics or oils, oleoresins and extracts derived from plants, leaves, flowers, fruits, and so forth, and combinations thereof. Non-limiting representative flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, watermelon, apricot, banana, melon, apricot, ume, cherry, raspberry, blackberry, tropical fruit, mango, mangosteen, pomegranate, papaya and so forth. Other potential flavors include a milk flavor, a butter flavor, a cheese flavor, a cream flavor, and a yogurt flavor; a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with cooling agents.

Flavors may also provide breath freshening properties, particularly the mint flavors when used in combination with cooling agents. These flavorings may be used in liquid or solid form and may be used individually or in admixture. Other useful flavorings include aldehydes and esters such as cinnamyl acetate, cinnamaldehyde, citral diethylacetal, dihydrocarvyl acetate, eugenyl formate, p- methylamisol, and so forth may be used. Generally any flavoring or food additive such as those described in CHEMICALS USED IN FOOD PROCESSING, Publication 1274, pp. 63-258 (National Academy of Sciences) may be used.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6- dimethyl-5-heptenal, i.e., melonal (melon), 2,6-dimethyloctanal (green fruit), and 2- dodecenal (citrus, mandarin), cherry, grape, strawberry shortcake, and mixtures thereof. These listings of flavorings are merely exemplary and are not meant to limit either the term "flavoring" or the scope of the disclosure generally.

In some embodiments, the flavoring may be employed in either liquid form and/or dried form. When employed in the latter form, suitable drying means such as spray drying the oil may be used. Alternatively, the flavoring may be absorbed onto water soluble materials, such as cellulose, starch, sugar, maltodextrin, gum arabic and so forth or may be encapsulated. The actual techniques for preparing such dried forms are well-known.

In some embodiments, the tabletop sweetener can be made to be similar to brown sugar. In such embodiments, compounds imparting brown notes can be added to the composition to make it taste more similar to brown sugar.

In some embodiments, the flavorings may be used in many distinct physical forms well- known in the art to provide an initial burst of flavor and/or a prolonged sensation of flavor. Without being limited thereto, such physical forms include free forms, such as spray dried, powdered, beaded forms, encapsulated forms, and mixtures thereof.

Suitable bulking agents include, but are not limited to maltodextrin (10 DE, 18 DE, or 5 DE), corn syrup solids (20 or 36 DE), sucrose, fructose, glucose, invert sugar, sorbitol, xylose, ribulose, mannose, xylitol, mannitol, galactitol, erythritol, maltitol, lactitol, isomalt, maltose, tagatose, lactose, inulin, glycerol, propylene glycol, polyols, polydextrose, fructooligosaccharides, cellulose and cellulose derivatives, and the like, and mixtures thereof. Additionally, granulated sugar (sucrose) or other caloric sweeteners such as crystalline fructose, other carbohydrates, or sugar alcohols can be used as a bulking agent due to their provision of good content uniformity without the addition of significant calories.

In one embodiment, the at least one bulking agent may be a bulking agent described in U.S. Patent No. 8,993,027. In another embodiment, the at least one bulking agent may be a bulking agent described in U.S. Patent No. 6,607,771. In another embodiment, the at least one bulking agent may be a bulking agent described in U.S. Patent No. 6,932,982.

In some embodiments, the tabletop sweetener composition may further comprise at least one anti-caking agent. As used herein the phrase "anti-caking agent" and "flow agent" refer to any composition which prevents, reduces, inhibits, or suppresses the at least one sweetener from attaching, binding, or contacting to another sweetener molecule. Alternatively, anti-caking agent may refer to any composition which assists in content uniformity and uniform dissolution. Non-limiting examples of anti-caking agents include cream of tartar, calcium silicate, silicon dioxide, microcrystalline cellulose (Avicel, FMC BioPolymer, Philadelphia, Pa.), and tricalcium phosphate. In one embodiment, the anti-caking agents are present in the tabletop sweetener composition in an amount from about 0.001 to about 3% by weight of the tabletop sweetener composition.

In some embodiments, the sweetener compositions of any of the preceding aspects and embodiments thereof are encapsulated using typical means for encapsulating flavor or fragrance compounds. Non-limiting examples of such technology are set forth in U.S. Patent Application Publication Nos. 2016/0235102, 2019/0082727, 2018/0369777, 2018/0103667, 2016/0346752, 2015/0164117, 2014/0056836, 2012/0027866, 2010/0172945, and 2007/0128234, as well as U.S. Patent Nos. 7,488,503, 6,416,799, 5,897,897, 5,786,017, 5,603,971, 4,689,235, 4,610,890, 3,704,137, 3,041,180, and 2,809,895. **EXAMPLES**

### Example 1 - Extraction and Characterization

Extract of *Siraitia grosvenorrii* extract (60 kg, light yellow liquid, 3.5% of mogroside V) obtained from Gui Lin Layn Natural Ingredients Corp. (Shanghai, China) was dissolved in 150 L de-ionized water and loaded on XAD macroporous resin. The resin was then eluted with water, 5% of EtOH in water and 95% of EtOH in water, and the last fraction enriched in mogrosides was collected as Frac 1 (5.0 kg). Frac 1 was further fractionized on a C18 flash chromatography column (Daiso ODS, 40-70 µm, 100*490 mm). After sample loading, the column was eluted with water, 10%, 20%, 25%, 30%, 40%, 50% ACN in water and 100% of ACN at a flow rate of 70 mL/min, and 16 fractions were collected (Frac. 35-50). Frac. 46 (49 g) was further purified via a C18 flash chromatography column (Daiso ODS, 40-70 µm, 100*490 mm, 26% ACN in water, 70 mL/min), a Sephadex LH-20 column (55*1500 mm, 18% ACN in water, 0.3 to 1.5 mL/min), and a preparative HPLC (YMC ODS, 5 µm, 10*250 mm, 14-21% ACN in water, 4.5 mL/min) to afford (1S,4R,9beta,11alpha,24R)-1-{[4-O-(beta-D-glucopyranosyl)-beta-D-glucopyranosyl]oxy}-11,25-dihydroxy-9,10,14-trimethyl-4,9-cyclo-9,10-secocholest-5-en-24-yl 2-O-beta-D-glucopyranosyl-beta-D-glucopyranoside (isomogroside IVE, Compound 11a) (50 mg, purity: 96%).

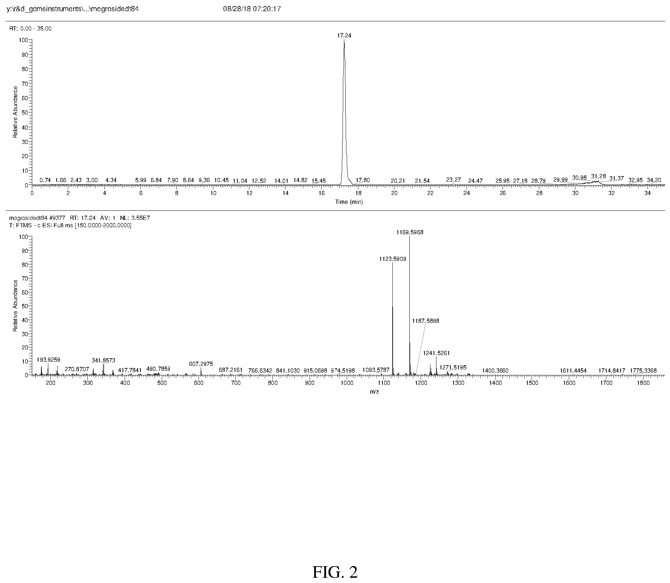

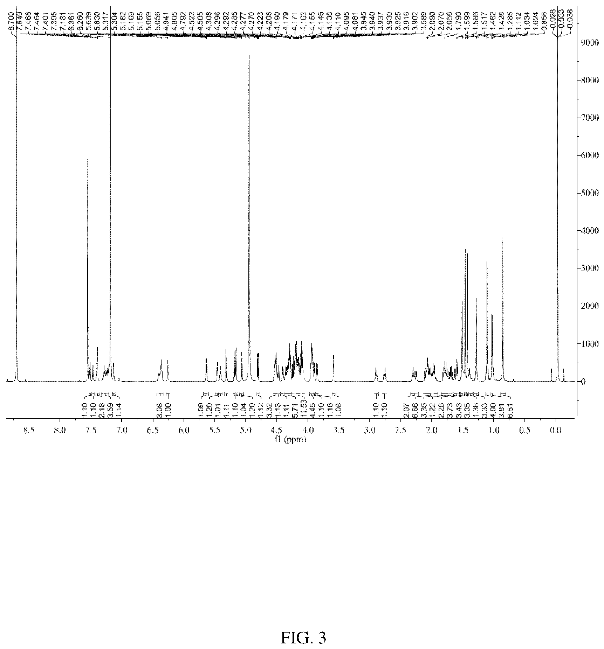

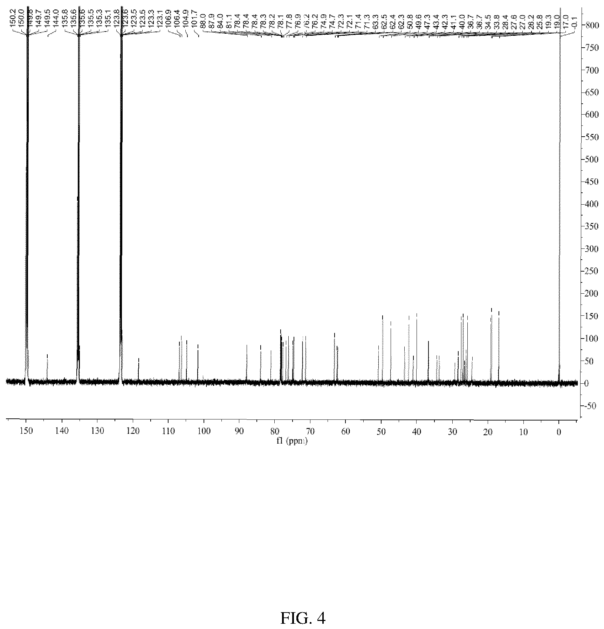

Referring to mass spectroscopy and NMR analysis shown in FIGS 2-4, the following structure was deduced for the compound:

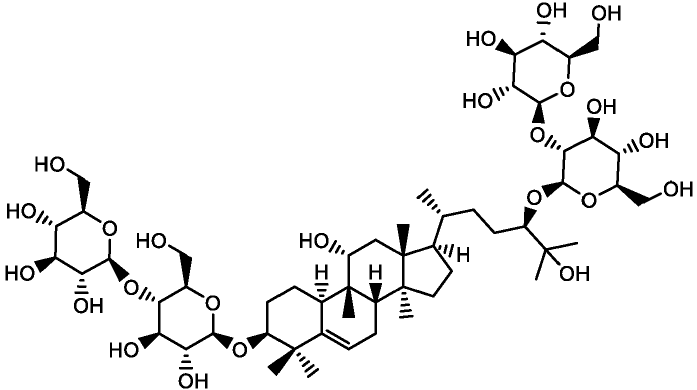

### Example 2 - Sensory Testing

Sensory testing was conducted by a group of about 5 panelists. A strong sweet taste, approximately 200-300 times sweeter than sucrose was perceived when a sample of the purified compound was tasted at a concentration of 250 ppm in a water base. The panelists rated the sweetness, licorice taste, and lingering aftertaste of Isomogroside IV_{E} in comparison to Mogroside V on a scale from 1 to 10. The results are shown in Table 1. As shown in Table 1, the panelists perceived Isomogroside IV_{E} to be somewhat sweeter than Mogroside V, and to have no statistically significant difference in licorice taste and lingering aftertaste.

**Table 1**

| Dosage (250 ppm) | Sweet | Licorice | Lingering |
|---|---|---|---|
| Mogroside V | 5.1 | 3.7 | 4.6 |
| Isomogroside IV_{E} | 7.0 | 4.5 | 5.4 |

## Claims

1. Use of a mogroside compound to sweeten a comestible composition or to enhance the sweetness of a comestible composition, wherein the mogroside compound is a compound of formula (1):

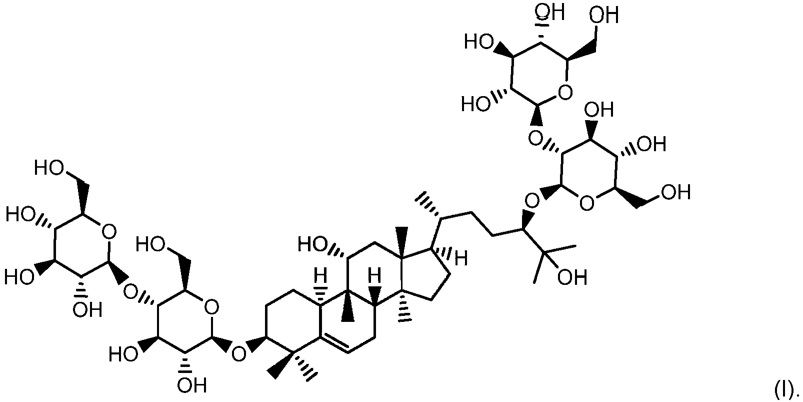

2. The use of claim 1, wherein the mogroside compound is present in the comestible composition at a concentration ranging from 1 ppm to 1000 ppm.

3. The use of claims 1 or 2, wherein the comestible composition comprises one or more additional sweeteners, preferably wherein the one or more additional sweeteners are selected from the group consisting of: sucrose, fructose, glucose, allulose, sugar alcohols; such as xylitol and erythritol; sucralose, aspartame, acesulfame potassium, cyclamate, steviol glycosides ; such as rebaudioside A, rebaudioside D, rebaudioside E, and rebaodioside M; other mogrosides; such as mogroside III, mogroside IV, mogroside V, siamenoside I, isomogroside V, mogroside IV_{E}, isomogroside IV, mogroside III_{E}, 11-oxomogroside V, and 1,6-α isomer of siamenoside I; and any combinations thereof.

4. The use of any one of claims 1 to 3, wherein the comestible composition comprises one of more flavor modifying compounds selected from the group consisting of: sweetness enhancing compounds, umami enhancing compounds, cooling enhancing compounds, sourness reducing compounds, bitterness blocking compounds, taste masking compounds, mouthfeel enhancing compounds, and combinations thereof.

5. The use of any one of claims 1 to 4, wherein the comestible composition is a food product, which optionally comprises a bulking agent, a starch, or a combination thereof.

6. The use of any one of claims 1 to 5, wherein the comestible composition is a beverage product, which optionally comprises an aqueous carrier.

7. A comestible composition, which comprises a mogroside compound of claim 1, and, optionally, a flavoring,
wherein the mogroside compound is present in the comestible composition at a concentration ranging from 5 ppm to 1000 ppm.

8. The comestible composition of claim 7, wherein the comestible composition comprises an aqueous carrier, preferably wherein the comestible composition is a beverage product, such as a packaged beverage product.

9. The comestible composition of claim 8, wherein the comestible composition comprises a bulking agent, a starch, or a combination thereof.

10. The comestible composition of claim 9, wherein the comestible composition is a food product, such as a packaged food product.

11. The comestible composition of any one of claims 7 to 10, wherein the comestible composition comprises one or more additional sweeteners selected from the group consisting of: sucrose, fructose, glucose, allulose, sugar alcohols; such as xylitol and erythritol; sucralose, aspartame, acesulfame potassium, cyclamate, steviol glycosides; such as rebaudioside A, rebaudioside D, rebaudioside E, and rebaodioside M; other mogrosides; such as mogroside III, mogroside IV, mogroside V, siamenoside I, isomogroside V, mogroside IV_{E}, isomogroside IV, mogroside III_{E}, 11-oxomogroside V, and 1,6-α isomer of siamenoside I; and any combinations thereof.

12. The comestible composition of any one of claims 7 to 11, wherein the comestible composition comprises one of more flavor modifying compounds selected from the group consisting of: sweetness enhancing compounds, umami enhancing compounds, cooling enhancing compounds, sourness reducing compounds, bitterness blocking compounds, taste masking compounds, mouthfeel enhancing compounds, and combinations thereof.

13. The comestible composition of any one of claims 7 to 12, wherein the comestible composition is not a naturally occurring composition.

## Patentansprüche

1. Verwendung einer Mogrosidverbindung zum Süßen einer essbaren Zusammensetzung oder zur Verstärkung der Süße einer essbaren Zusammensetzung, wobei es sich bei der Mogrosidverbindung um eine Verbindung der Formel (I) handelt:

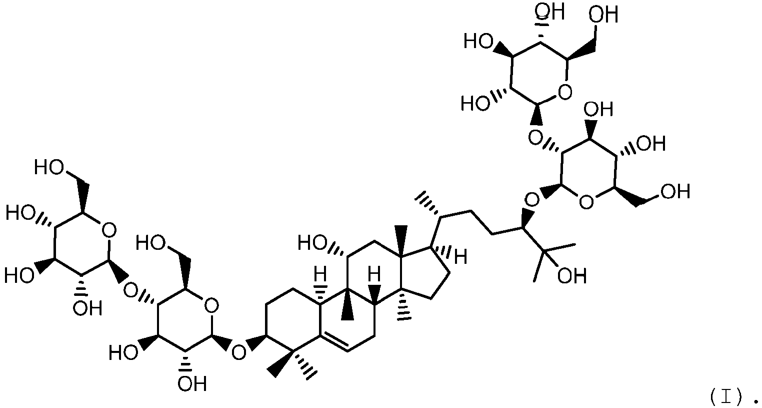

2. Verwendung nach Anspruch 1, wobei die Mogrosidverbindung in der essbaren Zusammensetzung in einer Konzentration im Bereich von 1 ppm bis 1000 ppm vorliegt.

3. Verwendung nach Anspruch 1 oder 2, wobei die essbare Zusammensetzung ein oder mehrere zusätzliche Süßungsmittel umfasst, wobei das eine oder die mehreren zusätzlichen Süßungsmittel vorzugsweise aus der aus den folgenden bestehenden Gruppe ausgewählt sind: Saccharose, Fructose, Glucose, Allulose, Zuckeralkohole, wie Xylit und Erythrit; Sucralose, Aspartam, Acesulfam-Kalium, Cyclamat, Steviolglycoside, wie Rebaudiosid A, Rebaudiosid D, Rebaudiosid E und Rebaudiosid M; andere Mogroside, wie Mogrosid III, Mogrosid IV, Mogrosid V, Siamenosid I, Isomogrosid V, Mogrosid IV_{E}, Isomogrosid IV, Mogrosid III_{E}, 11-Oxomogrosid V und das 1,6-α-Isomer von Siamenosid I; und beliebige Kombinationen davon.

4. Verwendung nach einem der Ansprüche 1 bis 3, wobei die essbare Zusammensetzung eine von mehreren geschmacksmodifizierenden Verbindungen umfasst, die aus der aus den folgenden bestehenden Gruppe ausgewählt sind: süßeverstärkende Verbindungen, umamiverstärkende Verbindungen, kühlungsverstärkende Verbindungen, säurereduzierende Verbindungen, bitterheitsblockierende Verbindungen, geschmacksmaskierende Verbindungen, das Mundgefühl verbessernde Verbindungen und Kombinationen davon.

5. Verwendung nach einem der Ansprüche 1 bis 4, wobei die essbare Zusammensetzung ein Lebensmittelprodukt ist, das gegebenenfalls einen Füllstoff, eine Stärke oder eine Kombination davon umfasst.

6. Verwendung nach einem der Ansprüche 1 bis 5, wobei die essbare Zusammensetzung ein Getränkeprodukt ist, das gegebenenfalls einen wässrigen Träger umfasst.

7. Essbare Zusammensetzung, die eine Mogrosidverbindung nach Anspruch 1 und gegebenenfalls einen Aromastoff umfasst,
wobei die Mogrosidverbindung in der essbaren Zusammensetzung in einer Konzentration im Bereich von 5 ppm bis 1000 ppm vorliegt.

8. Essbare Zusammensetzung nach Anspruch 7, wobei die essbare Zusammensetzung einen wässrigen Träger umfasst, wobei die essbare Zusammensetzung vorzugsweise ein Getränkeprodukt, wie etwa ein verpacktes Getränkeprodukt, ist.

9. Essbare Zusammensetzung nach Anspruch 8, wobei die essbare Zusammensetzung einen Füllstoff, eine Stärke oder eine Kombination davon umfasst.

10. Essbare Zusammensetzung nach Anspruch 9, wobei die essbare Zusammensetzung ein Lebensmittelprodukt, wie etwa ein verpacktes Lebensmittelprodukt, ist.

11. Essbare Zusammensetzung nach einem der Ansprüche 7 bis 10, wobei die essbare Zusammensetzung ein oder mehrere zusätzliche Süßungsmittel umfasst, die aus der aus den folgenden bestehenden Gruppe ausgewählt sind: Saccharose, Fructose, Glucose, Allulose, Zuckeralkohole, wie Xylit und Erythrit; Sucralose, Aspartam, Acesulfam-Kalium, Cyclamat, Steviolglycoside, wie Rebaudiosid A, Rebaudiosid D, Rebaudiosid E und Rebaudiosid M; andere Mogroside, wie Mogrosid III, Mogrosid IV, Mogrosid V, Siamenosid I, Isomogrosid V, Mogrosid IV_{E}, Isomogrosid IV, Mogrosid III_{E}, 11-Oxomogrosid V und das 1,6-α-Isomer von Siamenosid I; und beliebige Kombinationen davon.

12. Essbare Zusammensetzung nach einem der Ansprüche 7 bis 11, wobei die essbare Zusammensetzung eine von mehreren geschmacksmodifizierenden Verbindungen umfasst, die aus der aus den folgenden bestehenden Gruppe ausgewählt sind: süßeverstärkende Verbindungen, umamiverstärkende Verbindungen, kühlungsverstärkende Verbindungen, säurereduzierende Verbindungen, bitterheitsblockierende Verbindungen, geschmacksmaskierende Verbindungen, das Mundgefühl verbessernde Verbindungen und Kombinationen davon.

13. Essbare Zusammensetzung nach einem der Ansprüche 7 bis 12, wobei die essbare Zusammensetzung keine natürlich vorkommende Zusammensetzung ist.

## Revendications

1. Utilisation d'un composé mogroside pour édulcorer une composition comestible ou pour augmenter la sucrosité d'une composition comestible, dans laquelle le composé mogroside est un composé de formule (I) :

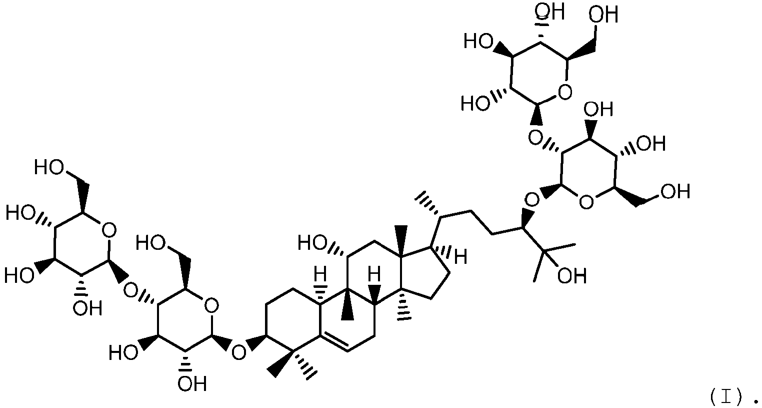

2. Utilisation selon la revendication 1, dans laquelle le composé mogroside est présent dans la composition comestible à une concentration allant de 1 ppm à 1 000 ppm.

3. Utilisation selon les revendications 1 ou 2, dans laquelle la composition comestible comprend un ou plusieurs édulcorants supplémentaires, de préférence dans laquelle le ou les édulcorants supplémentaires sont choisis dans le groupe constitué par : saccharose, fructose, glucose, allulose, alcools de sucre ; tels que le xylitol et l'érythritol ; sucralose, aspartame, acésulfame potassium, cyclamate, glycosides de stéviol ; tels que le rébaudioside A, le rébaudioside D, le rébaudioside E, et le rébaudioside M ; autres mogrosides ; tels que le mogroside III, le mogroside IV, le mogroside V, le siaménoside I, l'isomogroside V, le mogroside IV_{E}, l'isomogroside IV, le mogroside III_{E}, le 11-oxomogroside V et l'isomère 1,6-α du siaménoside I ; et toute combinaison de ceux-ci.

4. Utilisation selon l'une quelconque des revendications 1 à 3, dans laquelle la composition comestible comprend un ou plusieurs composés de modification d'arôme choisis dans le groupe constitué par : des composés améliorant la sucrosité, des composés améliorant l'umami, des composés améliorant le refroidissement, des composés réduisant l'aigreur, des composés bloquant l'amertume, des composés masquant le goût, des composés améliorant la sensation en bouche, et des combinaisons de ceux-ci.

5. Utilisation selon l'une quelconque des revendications 1 à 4, dans laquelle la composition comestible est un produit alimentaire, qui comprend éventuellement un agent de charge, un amidon, ou une combinaison de ceux-ci.

6. Utilisation selon l'une quelconque des revendications 1 à 5, dans laquelle la composition comestible est un produit de boisson, qui comprend éventuellement un support aqueux.

7. Composition comestible, qui comprend un composé mogroside selon la revendication 1, et, éventuellement, un arôme,
dans laquelle le composé mogroside est présent dans la composition comestible à une concentration allant de 5 ppm à 1 000 ppm.

8. Composition comestible selon la revendication 7, dans laquelle la composition comestible comprend un support aqueux, de préférence dans laquelle la composition comestible est un produit de boisson, tel qu'un produit de boisson emballé.

9. Composition comestible selon la revendication 8, dans laquelle la composition comestible comprend un agent de charge, un amidon, ou une combinaison de ceux-ci.

10. Composition comestible selon la revendication 9, dans laquelle la composition comestible est un produit alimentaire, tel qu'un produit alimentaire emballé.

11. Composition comestible selon l'une quelconque des revendications 7 à 10, dans laquelle la composition comestible comprend un ou plusieurs édulcorants supplémentaires choisis dans le groupe constitué par : saccharose, fructose, glucose, allulose, alcools de sucre ; tels que le xylitol et l'érythritol ; sucralose, aspartame, acésulfame potassium, cyclamate, glycosides de stéviol ; tels que le rébaudioside A, le rébaudioside D, le rébaudioside E, et le rébaudioside M ; autres mogrosides ; tels que le mogroside III, le mogroside IV, le mogroside V, le siaménoside I, l'isomogroside V, le mogroside IV_{E}, l'isomogroside IV, le mogroside III_{E}, le 11-oxomogroside V et l'isomère 1,6-α du siaménoside I ; et toute combinaison de ceux-ci.

12. Composition comestible selon l'une quelconque des revendications 7 à 11, dans laquelle la composition comestible comprend un ou plusieurs composés de modification d'arôme choisis dans le groupe constitué par : des composés améliorant la sucrosité, des composés améliorant l'umami, des composés améliorant le refroidissement, des composés réduisant l'aigreur, des composés bloquant l'amertume, des composés masquant le goût, des composés améliorant la sensation en bouche, et des combinaisons de ceux-ci.

13. Composition comestible selon l'une quelconque des revendications 7 à 12, dans laquelle la composition comestible n'est pas une composition naturelle.
